# DEMANDE DE BREVET EUROPEEN

(11) **EP 1 618 912 A1**
(43) Date de publication de la demande: **25.01.2006**
(21) Numéro de dépôt: 05356122.1
(22) Date de dépôt: 18.07.2005
(51) Int. Cl.: A61M 16/08

(54) **Embout pour masque à oxygène**

(30) Priorité: 19.07.2004 FR 0407978
(71) Demandeur: Ghiron, Fabrice, 38240 Meylan (FR); Hercule, Jean-Pierre, 69009 Lyon (FR); Wasylkiewicz, Jacek, 69500 Bron (FR)
(72) Inventeur: Ghiron, Fabrice, 38240 Meylan (FR); Hercule, Jean-Pierre, 69009 Lyon (FR); Wasylkiewicz, Jacek, 69500 Bron (FR)
(74) Mandataire: Schouller, Jean-Philippe

(57) **Abrégé**

Ce masque à oxygène (2) comprend un corps (4) destiné à recouvrir le nez et la bouche d'un patient, ainsi qu'un embout (6) propre à être solidarisé par rapport au corps (4), cet embout (6) comprenant des moyens (6₅) de connexion à une arrivée d'oxygène (8).

Cet embout (6) comprend également des moyens (6₆, 6₇) d'évacuation du gaz expiré par le patient, qui sont propres à être raccordés à un appareil (14) de détermination d'au moins un paramètre respiratoire du patient.

## Description

La présente invention concerne un embout pour masque à oxygène, un masque à oxygène équipé d'un tel embout, ainsi qu'un ensemble de surveillance respiratoire comprenant un tel masque.

Un masque à oxygène se compose, de façon classique, d'un corps souple, réalisé par exemple en poly-(chlorure de vinyle), ou PVC. Ce corps, qui recouvre en service la bouche et le nez du patient, reçoit un embout amovible, qui est notamment solidarisé par coopération de formes. Cet embout est muni d'un conduit d'arrivée d'oxygène, de forme appropriée, permettant l'alimentation du patient.

Un masque à oxygène, du type décrit ci-dessus, trouve son application dans de nombreuses circonstances.

Ainsi, il est tout d'abord utilisé en salle de réveil, à savoir après que le patient a été soumis à une anesthésie générale. Il est également possible de faire appel à un tel masque dans différentes pathologies respiratoires nécessitant un apport en oxygène et une surveillance respiratoire par capnographie. Enfin, on tire parti de ce masque à oxygène dans le cadre d'interventions chirurgicales, qui sont réalisées sous ventilation spontanée.

On conçoit que l'alimentation en oxygène par l'intermédiaire de ce masque s'accompagne d'une surveillance respiratoire du patient. Ceci permet de s'affranchir de tout risque d'accident, susceptible de se révéler dramatique.

Dans cette optique, il est tout d'abord connu de contrôler le taux de saturation du sang en oxygène, par exemple par un capteur infrarouge placé au bout du doigt du patient. Cette solution ne s'avère cependant pas suffisamment réactive, en particulier en cas d'une baisse sensible de la fréquence respiratoire du patient.

Il est également possible de mettre en oeuvre cette surveillance respiratoire, par le personnel soignant lui-même. Cependant, cette solution alternative est peu satisfaisante, en termes de sécurité. De plus, elle nécessite l'emploi d'une main d'oeuvre importante et relativement qualifiée.

Ceci étant précisé, l'invention se propose de remédier à l'ensemble des inconvénients de l'art antérieur évoqués ci-dessus.

A cet effet, elle a pour objet un embout pour masque à oxygène, propre à être solidarisé par rapport à un corps de ce masque, cet embout comprenant des moyens de connexion, propres à être raccordés à des moyens de fourniture d'oxygène, caractérisé en ce que cet embout comprend également des moyens de prélèvement du gaz expiré par le patient, ces moyens de prélèvement étant propres à être raccordés à un appareil de détermination d'au moins un paramètre respiratoire du patient.

L'invention a également pour objet un masque à oxygène comprenant un corps destiné à recouvrir le nez et la bouche d'un patient, ainsi qu'un embout propre à être solidarisé par rapport au corps, notamment par emmanchement, caractérisé en ce que l'embout est tel que défini ci-dessus.

L'invention a enfin pour objet un ensemble de surveillance respiratoire comprenant :
- un masque à oxygène qui est tel que défini ci-dessus ;
- des moyens de fourniture d'oxygène ;
- des moyens de raccordement entre ces moyens de fourniture d'oxygène et les moyens de connexion appartenant au masque ;
- des moyens de détermination d'au moins un paramètre respiratoire du patient ; et
- des moyens de raccordement entre ces moyens de détermination et les moyens de prélèvement appartenant au masque.

L'invention sera mieux comprise et d'autres avantages de celle-ci apparaîtront plus clairement à la lumière de la description qui va suivre d'un ensemble de surveillance respiratoire conforme à son principe, donnée uniquement à titre d'exemple non limitatif et faite en référence aux dessins annexés, dans lesquels :
- la figure 1 est une vue schématique, illustrant un ensemble de surveillance respiratoire conforme à l'invention ;
- la figure 2 est une vue de côté, à plus grande échelle, illustrant un embout appartenant à un masque à oxygène qui équipe l'ensemble de surveillance respiratoire de la figure 1 ; et
- la figure 3 est une vue de face, illustrant l'embout de la figure 2.

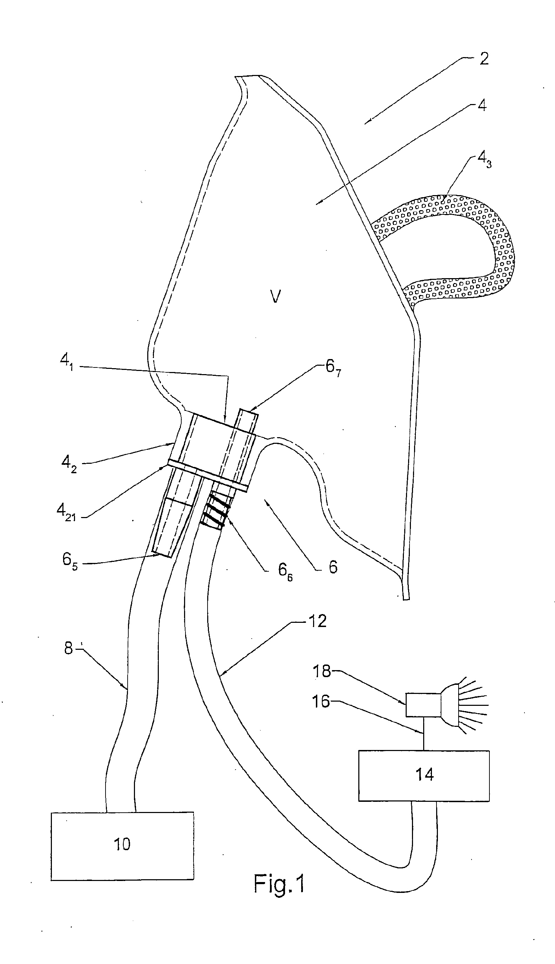

L'ensemble de surveillance respiratoire, représenté sur la figure 1, comprend tout d'abord un masque à oxygène, désigné dans son ensemble par la référence 2. Ce dernier comporte, de façon connue en soi, un corps souple 4, réalisé notamment en PVC, ou poly-(chlorure de vynile). Ce corps souple 4 est destiné à recouvrir, en service, le nez et la bouche du patient.

Dans sa partie antérieure, en référence à sa position portée, le corps souple est creusé d'un orifice 4₁, à partir duquel s'étend un col 4₂, dirigé à l'opposé du volume intérieur V du masque. Comme on le verra de façon plus détaillée dans ce qui suit, ce col 4₂ est destiné à la réception d'un embout 6.

Enfin, le corps 4 est pourvu d'un lien élastique 4₃, propre à être disposé autour de la tête du patient, afin d'assurer la tenue de l'ensemble du masque 2.

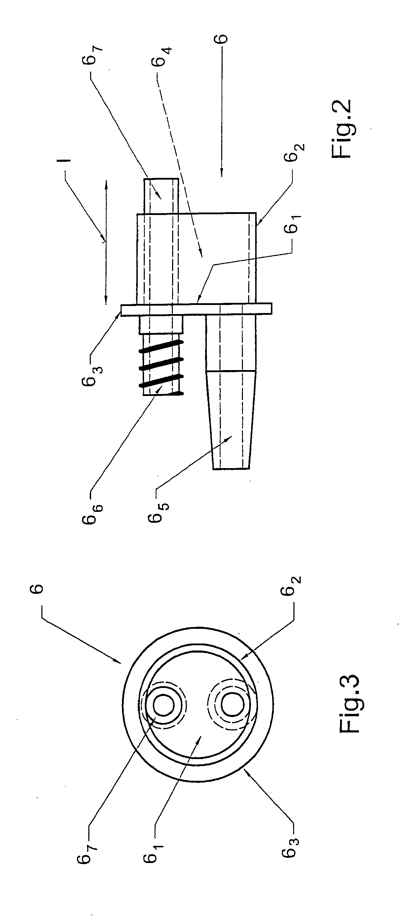

En référence notamment aux figure 2 et 3, cet embout 6 comprend une embase 6₁, à partir de laquelle s'étend un fût cylindrique 6₂, destiné à être emmanché dans le col 4₂. Cette embase est en outre munie d'une collerette 6₃, destinée à prendre appui contre l'extrémité libre 4₂₁ du col 4₂, cette extrémité libre formant ainsi un siège pour l'embout 6.

On notera par ailleurs que le fût 6₂, dont les dimensions axiales correspondent sensiblement à celles du col 4₂, définit une chambre notée 6₄. Du côté extérieur, opposé au volume V, il est prévu un conduit 6₅ débouchant dans cette chambre 6₄. Ce conduit 6₅ est destiné à être raccordé avec une ligne 8, visible sur la figure 1, qui est mise en communication avec des moyens de fourniture d'oxygène, notés 10, qui sont connus en tant que tels.

Au voisinage du conduit 6₅, du côté extérieur de l'embout, il est en outre prévu un raccord fileté 6₆, propre à coopérer par vissage avec un tuyau souple 12. Ce dernier est lui-même mis en communication avec un appareil 14, de type connu en soi, qui est propre à déterminer en temps réel au moins un paramètre respiratoire du patient. Cet appareil 14 est par ailleurs relié, par l'intermédiaire d'une ligne 16, à une alarme 18, qui est par exemple de type sonore.

En revenant en particulier à la figure 2, le raccord fileté 6₆ débouche dans un tube 6₇, s'étendant depuis l'embase 6₁ en direction du volume intérieur V. Il est à noter que ce tube 6₇, qui est propre à évacuer le gaz expiré par le patient, débouche directement dans le raccord 6₆, en ce sens qu'il n'est pas mis en communication avec la chambre 6₄.

De plus, comme le montrent notamment les figures 1 et 2, le tube de prélèvement 6₇ fait saillie, en direction du volume intérieur précité V, à la fois par rapport au fût 6₂ de l'embout 6 et par rapport à l'orifice 4₁ du corps souple 4. De façon avantageuse, la longueur l du tube 6₇ est comprise entre 15 et 35 mm, en étant notamment voisine de 20 mm.

Cette configuration du tube de prélèvement est avantageuse, puisqu'elle permet d'éviter tout mélange entre les flux respectifs, formés par l'oxygène et le gaz expiré par le patient. A titre de variante non représentée, le conduit d'alimentation en oxygène peut être prolongé par un tube d'alimentation, en saillie vers le volume intérieur V, alors que le tube de prélèvement est raccourci, voire supprimé.

L'utilisation de l'ensemble de surveillance respiratoire, décrit ci-dessus, va maintenant être explicitée.

Il s'agit tout d'abord d'administrer de l'oxygène au patient, de façon classique. Cette opération est réalisée grâce aux moyens de fourniture 10 qui délivrent cet oxygène vers le volume intérieur V, via la ligne 8 et le conduit 6₅.

De façon concomitante, le gaz expiré par le patient est dirigé vers l'appareil d'analyse 14, par l'intermédiaire successivement du tube de prélèvement 6₇, du raccord 6₆ et du tuyau souple 12. Cet appareil 14 est notamment pourvu d'un calculateur propre à déterminer, entre autres, la fréquence respiratoire du patient, ainsi que le taux de dioxyde de carbone CO₂ présent dans le gaz expiré par ce patient.

Si les valeurs instantanées ainsi déterminées se situent en dehors d'une plage prédéterminée, l'alarme 18 est activée, ce qui permet au personnel soignant d'intervenir sur le patient. A titre d'exemple non limitatif, cette alarme 18 est activée si la fréquence respiratoire descend au-dessous de 6 par minute et/ou si le taux de CO₂ dans le gaz expiré devient supérieur à 30%.

L'invention permet d'atteindre les objectifs précédemment mentionnés. En effet, elle garantit une surveillance satisfaisante du patient, en particulier dans la mesure où elle permet de détecter toutes les variations substantielles des paramètres respiratoires de ce dernier. Par conséquent, les risques d'accident se trouvent sensiblement éliminés.

De plus, l'invention confère une grande précision à la détermination des paramètres respiratoires du patient. A cet égard, il est tout particulièrement avantageux de disposer, sur un même embout, à la fois les moyens d'arrivée d'oxygène et les moyens d'évacuation du gaz expiré par le patient.

## Revendications

1. Embout (6) pour masque à oxygène (2), propre à être solidarisé par rapport à un corps (4) de ce masque (2), cet embout (6) comprenant des moyens (6₅) de connexion, propres à être raccordés à des moyens (10) de fourniture d'oxygène, **caractérisé en ce que** cet embout (6) comprend également des moyens (6₆, 6₇) de prélèvement du gaz expiré par le patient, ces moyens de prélèvement étant propres à être raccordés à un appareil (14) de détermination d'au moins un paramètre respiratoire du patient.

2. Embout pour masque à oxygène selon la revendication 1, **caractérisé en ce que** les moyens de connexion (6₅) et les moyens de prélèvement (6₆, 6₇) s'étendent à partir d'une embase (6₁) dudit embout (6), cette embase étant propre à venir en appui contre un siège (4₂₁) du corps (4).

3. Embout pour masque à oxygène selon la revendication 2, **caractérisé en ce que** les moyens de prélèvement comprennent un tube de prélèvement (6₇), s'étendant à partir de l'embase (6₁) en direction d'un volume intérieur (V) du corps (4), ainsi qu'un raccord de prélèvement (6₆) s'étendant à partir de l'embase (6₁) à l'opposé dudit volume intérieur (V), ce raccord de prélèvement débouchant directement dans le tube de prélèvement.

4. Embout pour masque à oxygène selon la revendication 3, **caractérisé en ce que** l'embout (6) comprend un fût (6₂) propre à prendre appui contre des parois d'un col (4₂) du corps (4), le tube de prélèvement (6₇) faisant saillie au-delà du fût (6₂), en direction du volume intérieur (V).

5. Embout pour masque à oxygène selon la revendication 3 ou 4, **caractérisé en ce que** le raccord de prélèvement (6₆) est disposé à côté d'un conduit (6₅) de connexion auxdits moyens de fourniture d'oxygène (10).

6. Embout pour masque à oxygène selon l'une quelconque des revendications 3 à 5, **caractérisé en ce que** le tube de prélèvement (6₇) présente une dimension axiale, ou longueur (l), qui est comprise entre 15 et 35 mm, notamment voisine de 20 mm.

7. Masque à oxygène (2) comprenant un corps (4) destiné à recouvrir le nez et la bouche d'un patient, ainsi qu'un embout (6) propre à être solidarisé par rapport au corps (4), notamment par emmanchement, **caractérisé en ce que** l'embout (6) est conforme à l'une quelconque des revendications précédentes.

8. Ensemble de surveillance respiratoire comprenant :
- un masque à oxygène (2) conforme à la revendication précédente ;
- des moyens (10) de fourniture d'oxygène ;
- des moyens (8) de raccordement entre ces moyens de fourniture d'oxygène (10) et les moyens de connexion (6₅) appartenant au masque (2) ;
- des moyens (14) de détermination d'au moins un paramètre respiratoire du patient ; et
- des moyens (12) de raccordement entre ces moyens de détermination (14) et les moyens de prélèvement (6₆, 6₇) appartenant au masque (2).

9. Ensemble de surveillance respiratoire selon la revendication 8, **caractérisé en ce que** cet ensemble comprend également une alarme (18) propre à être activée si une valeur du ou de chaque paramètre respiratoire, déterminée par les moyens de détermination (14), se situe en dehors d'une plage prédéterminée.

10. Ensemble de surveillance respiratoire selon la revendication 8 ou 9, **caractérisé en ce qu'**un paramètre respiratoire est la fréquence respiratoire du patient, et/ou le taux de dioxyde de carbone (CO₂) présent dans le gaz expiré par le patient.
